# EUROPEAN PATENT APPLICATION

(11) **EP 0 685 558 A1**
(43) Date of publication of application: **06.12.1995**
(21) Application number: 94108193.7
(22) Date of filing: 27.05.1994
(51) Int. Cl.: C12N 15/00, A01K 67/027, C12N 15/12, C07K 14/47, C12N 15/90

(54) **Transgenic animals lacking proteolipid protein and method of making such animals**

(71) Applicant: BAYER AG, 51368 Leverkusen (DE)
(72) Inventor: Stoffel, wilhelm Prof. Dr., D 50933 Köln (DE); Boison,Detlev Dr., D 50937 Köln (DE)

(57) **Abstract**

Proteolipid protein and its isoprotein DM20, the two splice products of the PLP gene, are the two major membrane proteins of CNS myelin. For structural and functional analysis during myelination we replaced the PLP gene in mouse ES-cells by a mutated PLP targeting construct (deletion of 105 bp at the 3' end of exon III) with an antisense (as) oriented neo-gene in intron III, (plp⁻/dm20⁺/asneo). Brain RNAs of transgenic mice unexpectedly are devoid of PLP and DM20 mRNAs, PLP and DM20 isoproteins are completely missing in myelin. The plasma membrane processes of oligodendrocytes of the PLP-deficient mouse are spirally wrapped around axons of large diameter but have lost their compacted structure, smaller axons are non- or hypomyelinated. Young transgenic mice manifest no severe loss of CNS functions, although the conductance velocity is reduced by one half in PLP-deficient mice. These results define PLP as an essential membrane component responsible for the highly ordered myelin sheath of CNS axons.

## Description

### Introduction

The myelin sheaths of axons of the CNS provide insulation, energy conservation and compaction, and allow for a rapid information transmission by saltatory conductance. Proteolipid protein (PLP;30 kD) and its isoform DM20 (26 kD) are the most abundant integral membrane protein constituents of myelin in central nervous system. PLP is expressed in oligodendrocytes immediately alter their differentiation from their progenitor O2A cell, coinciding in time with peak myelin synthesis between postnatal day 10 to 30 in rodents. In contrast to PLP, DM20 transcripts have been detected by PCR as early as embryonal day 10. The topology of these extremely hydrophobic proteins in the lipid bilayer of myelin has been assessed biochemically. Six of its cysteine thiols are modified posttranslationally by acylation which render the protein even more hydrophobic. The functional status of the cysteine residues strongly suggests a four transmembrane helix model of PLP, also based on theoretical considerations. The PLP amino acid sequence, Stoffel and Hillen, (1983), Hoppe-Seyler's Zeitschr. Physiol. Chem. 364, has been confirmed by the nucleotide sequence of its cloned cDNA, and its gene organization and locus on the X-chromosome elucidated. The PLP gene spans 17,400 bp from its transcription start to the polyadenylation signal including appr. 5 kb 5' upstream regulatory sequence. The coding sequence of PLP is distributed over seven exons. PLP and DM20 are encoded by the same gene, DM20 being an alternative splice product of the PLP primary transcripts by usage of a cryptic splice site within exon III. In the mature DM20 transcript the 3' terminal 105 bases of exon III of PLP are deleted.

The PLP structure is extremely conserved during evolution. 99-100% amino acid identity can be found among mammalian species. A comparison of the primary structure of PLP in the CNS between lower classes (birds and amphibia) and humans reveals numerous conservative substitutions leaving, however, cysteine and charged amino acid residues in identical positions. This suggests their corresponding positions in the tertiary structure and identical functions of PLP in the myelin membrane. The highly conserved PLP structure may explain the low mutation tolerance.

Point mutations of PLP cause lethal dysmyelinoses in mouse, rat, dog, and human. In the jimpy mouse an A to G transition in the splice acceptor site of intron IV leads to the deletion of exon V accompanied by a frame shift of the ORF (Morello et al., (1986) EMBO J. 5,3489-3493; Nave et al., (1987), Neurochem. 49, 1873-1877. In the myelin-deficient (md) rat Thr⁷⁵ is mutated to Pro in the second transmembranal α-helix (Boison and Stoffel, (1989), EMBO J. 8,3295-3302. Further point mutations have been described in the jimpy^{msd} mouse, in the rumpshaker mouse, in the shaking pup (dog) and in several cases of the human inherited form of sudanophilic leukodystrophy, type Pelizaeus-Merzbacher. The pleiotropic phenotype is due to a total apoptosis of oligodendrocytes which leads to the premature death of the affected individuum by the end of the myelination period in all these cases except rumpshaker. The latter mutant shows hypomyelination without premature cell death of oligodendrocytes.

Up to now a molecular understanding of the death of the oligodendrocyte due to these fatal point mutations is missing.

Besides being an essential structural constituent of the CNS myelin membrane other functions have been assigned to PLP and DM20: PLP as an ion channel-forming protein or as an adhesive pore, and DM 20, transiently expressed during the fetal period, as a differentiation factor.

Recently a proteolipid gene family has been described, with its DM members containing regions with similarities to channel-forming segments of the nicotinic acetylcholine receptor and the glutamate receptor. Additionally, palmitoylated cytosolic segments of PLP/DM20 show a high degree of homology with palmitoylated segments of transmembrane proteins, e.g. rhodopsin and β2-adrenergic receptor (O'Dowd et al., 1989) coupled with G-proteins.

Gene targeting in mouse embryonic stem cells (Mansour et al., 1988) is a powerful method to determine the in vivo function of specific proteins, in our case the dissociation of PLP-function(s) from DM20-function(s). The PLP locus is well suited for this kind of functional dissociation because the PLP expression is confined mainly to one specific cell type, the oligodendrocyte, which differentiates postnatally. The X-chromosomal location facilitates the examination of replacement events in male ES cells. We have used homologous recombination in embryonic stem cells to replace the endogenous PLP gene on the X-chromosome with specific PLP and DM20 constructs, carrying a neomycin resistance gene in antisense orientation within intron III. With this invention we demonstrate that mice homozygous and heterorygous for this gene replacement display striking abnormalities in central myelination.

Until now, no valid models are available which mimic the pathology of multiple sclerosis. This prevents a straight forward development of drugs for this disease. The present invention provides a transgenic "knock-out" mouse with a defect myelinisation process. The morphological changes of myelinised fibers are evident alter histological examination of the respective nervous tissues. This transgenic mouse represents a new approach in the direction towards an animal model of multiple sclerosis. It most likely provides an appropriate tool for the investigation of malfunctions in neuronal myelinisation and of the subsequent effects on animal physiology and behaviour. Possibly, similar events might be involved in the morphological and molecular alterations characteristic of Alzheimer's disease and other neurodegenerative pathologies and could thus be covered by the same animal model. Moreover, this "knock-out" mouse might be relevant for the investigation of diseases accompanied by impairments of nerve conductance velocities and by an affected CNS metabolism.

The transgenic mouse provides a unique tool to find therapeutically relevant new targets, to screen novel substances in drug finding programs, and to test a variety of parameters derived from Behavioural Pharmacology, Physiology and Histology.

### Results

### Mutagenesis of the X-chromosomal PLP Locus

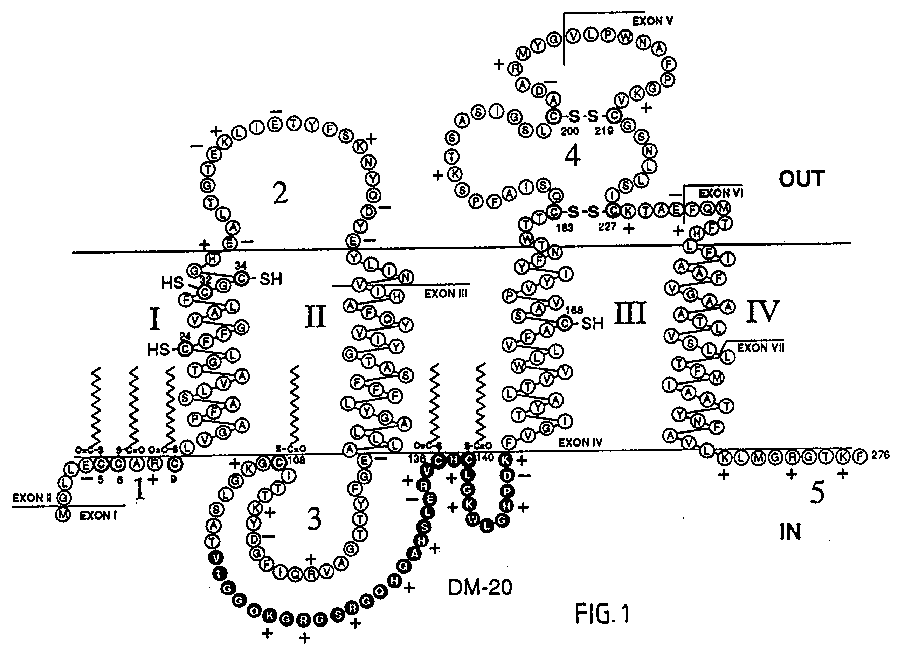

A promising way to define the function of PLP and DM20 either as structural components in the highly ordered, compacted multilayer membrane of CNS or as putative regulatory proteins during embryogenesis and myelination is the separate in vivo expression of PLP and DM20 in transgenic mice created by homologous recombination in embryonic stem cells. A 105 bp deletion in exon III of the PLP gene is contained within the alternative splice product DM20 devoid of a 35 amino acid sequence within the large cytosolic loop, Figure 1. This leads also to the loss of two out of six thioacylation sites present in PLP. Figure 1 shows the putative topology of PLP and DM20 in the myelin lipid bilayer and indicates the sequence missing in DM20.

We introduced subtle mutations into the locus of the PLP gene: a 105 bp deletion in exon III leading to the exclusive expression of DM20 and a two basepair substitution within the cryptic splice site of exon III, thus prohibiting splicing for the DM20 transcripts (Table 1), and leading to the exclusive expression of PLP.

Three methods have been described for the introduction of small subtle non-selectable mutations into ES cells via homologous recombination: DNA micro-injection (Zimmer and Gruss, P. (1989), Nature 338, 150-153, coelectroporation (Davis, A.C., Wims, M., and Bradley, A. (1992), Mol. Cell. Biol. 12,2769-2776, and the hit-and-run procedure (Hasty, P. Ramirez-Solis, R., Krumlauf, R., and Bradley, A. (1991), Nature 350,243-246; Valancius, V., and Smithies, O. (1991), Mol. Cell. Biol. 11, 1402-1408.

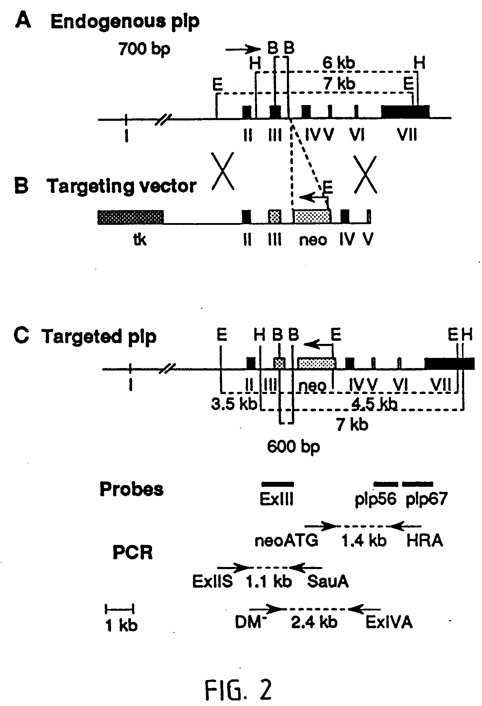

We applied a different strategy to introduce subtle mutations. Our assumption was that a neo cassette, introduced as a selection marker in antisense orientation into an intron of the gene to be targeted, is spliced together with the intron during RNA processing, thus leaving in our case the processed transcripts of DM20 and PLP respectively, containing only the desired mutations. Figure 2 shows our strategy to target the PLP locus in mouse embryonic stem cells.

We used a PLP clone derived from a genomic balb/c mouse library containing 6.7 kb of the PLP gene spanning parts of intron I to exon V for the construction of the replacement vectors pPLPMut25^{h}asneotk, pPLPDel25^{h}asneotk, and pPLPwt25^{h}asneotk for gene targeting by homologous recombination (Mansour, et al., (1988). Table 1 lists the respective sequences of exon III. The two mutagenized forms of exon III and wt-exon III were used for further construction of the targeting vectors. Transcriptionally competent cassettes of the Herpes simplex virus thymidine kinase (tk) gene and the bacterial neomycin resistance gene (neo) were introduced for positive-negative selection (Mansour S., et al., (1988), Nature 336, 348-352. The position of the neo-cassette in intron III with antisense orientation to the PLP gene is approximately 550 bp distant from the splice donor and 350 bp from the acceptor sites. The final targeting constructs are shown in Figure 2. They contain a total of 6.7 kb of homology to the targeting locus and have a blunt 3' end with 100% PLP gene homology. The cloning strategy of the targeting constructs is described under "Experimental Procedures". The linearized replacement vectors pPLPMut25^{h}asneotk, pPLPDel25^{h}asneotk, and pPLPwt25^{h}asneotk were introduced into recipient E14-ES cells by electroporation. ES cell clones were analyzed for targeting events after growing in double selection medium on neomycin-resistant mouse embryonic fibroblast monolayers. Approximately 2000 resistant clones per 10⁷ electroporated cells survived the G418 selection. By double selection with GANC we obtained a three- to fivefold enrichment.

### Genotyping of ES Cells and Mice Homozygous for the Targeted and Replaced Plp-Allele

Targeted ES cell clones were analyzed for homologous and heterologous recombination events by Southern analysis and polymerase chain reaction (PCR) assays.

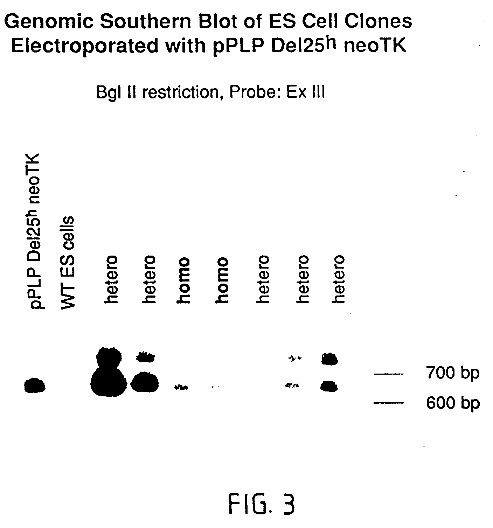

For analysis of the replacement event pooled DNA of single ES cell clones (3 clones per pool) was restricted with EcoRI and hybridized with the external plp56 and the plp67 probes locating sequences downstream of the 3' end of the targeting construct, Figure 3A. DNA of pooled clones marked with an asterisk yielded a 4.5 kb EcoRI fragment, indicating the correctly integrated replacement vector, containing an additional EcoRI site within the neo-cassette, in addition to the 7.0 kb wild type EcoRI fragment derived from the PLP gene of nontargeted cells and mouse embryonic fibroblasts. Individual clones from positive pools were analyzed in the same way by hybridization analysis, proving that one clone out of a pool of three is correctly targeted. In addition the correct structure of the mutated exon III was controlled for the presence of the DM20 deletion in case of plp⁻/dm20⁺/asneo clones by using a BglII restriction, cutting 5' and 3' of the DM20 deletion (Figure 3). The expected 600 bp DM20-specific fragment versus a 700 bp PLP-specific fragment was visualized by hybridization with an internal exon III probe (BamHI/HindIII fragment, containing exon III). Positive clones were also analyzed by PCR using primers neoATG (neo-box specific) and HRA (lying outside the targeting construct) for detection of the 1.4 kb indicative length for the homologous integration of the neo-box, and primers ExIIS/SauA, lying on either side of the DM20 deletion and giving rise to 1.2 and 1.1 kb amplification products for PLP and DM20, respectively. The allele-specific PCR primers DM⁺ and DM⁻ paired with SauA or ExIVA were used to probe for the existence of a mutated alternative splicing site in exon III of pPLPMut25^{h}asneotk derived clones (data not shown). Pursuing this strategy we obtained a total of five plp⁺/dm20⁻/asneo clones and only one plp⁻/dm20⁺/asneo clone due to a high degree of mismatch repair during homologous recombination, in addition to a plp⁺/dm20⁺/asneo control clone.

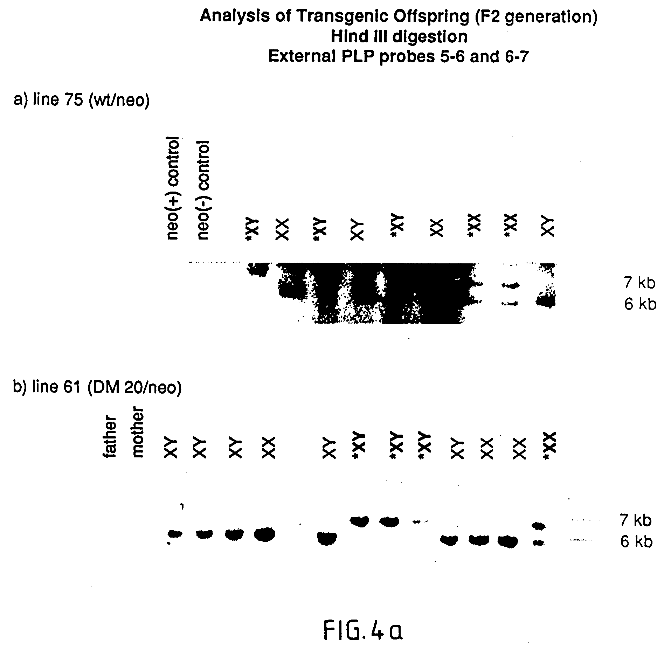

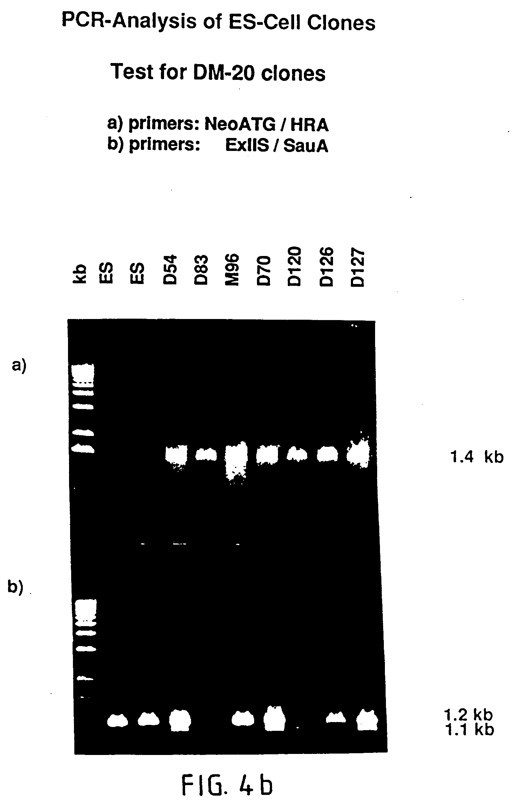

Mice homozygous for the targeted plp allele were generated by injection of the correctly targeted ES cell clones into blastocysts from either C57 Bl/6J or CD-1 female mice to generate germ line chimeras. Male mice showing coat color chimerism have been checked by tail DNA analysis and Southern blotting of the EcoRI digests and hybridization with the external plp56 and plp67 probes for the presence of the desired alleles on DNA level. Most coat color chimeras also proved to be highly chimeric on DNA level. These animals have been used for test breeding with either C57Bl/6J or CD1 females to determine the germline contribution of the ES cells. Thus two transgenic lines have been established, line 61, C57Bl/6J-derived, in which the plp locus is replaced by the DM20 construct of pPLPDel25^{h}asneotk and line 75, CD-1-derived, containing the plp⁺/dm20⁺ allele in conjunction with an antisense oriented neo-box in intron III. Heterozygous progeny with the transmitted mutation were crossed with wild type males giving rise to affected males which were bred with heterozygous females to establish homozygous mutant lines of plp⁻ /dm20⁺/asneo and plp⁺/dm20⁺/asneo mice. Figure 4 examplifies the analysis of offspring of the F2 generation of line 61, plp⁻/dm20⁺/asneo, probing for the homologous integration of the neo box with a HindIII digestion in a Southern blot (Figure 4A) and for the DM20 deletion with PCR-primers ExIIIS/SauA, lying on either side of the mutation, Figure 4B.

### Analysis of CNS mRNA and Myelin Proteins and Lipids of plp⁻/dm20⁺/asneo and plp⁺/dm20⁺/asneo Transgenic Mice

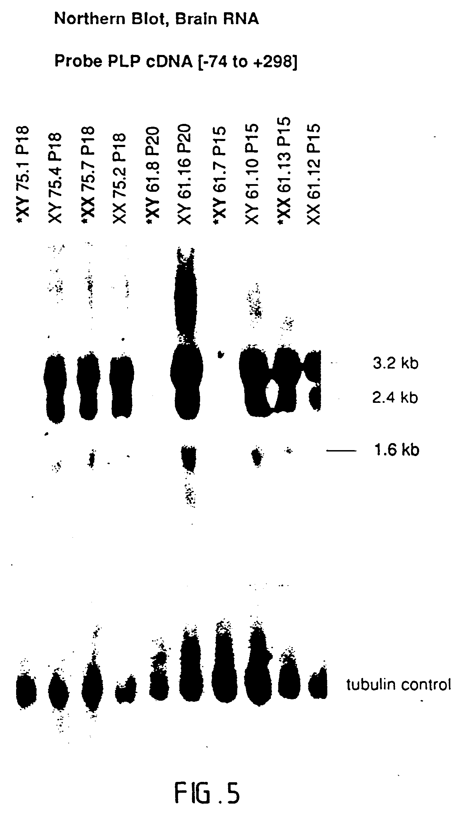

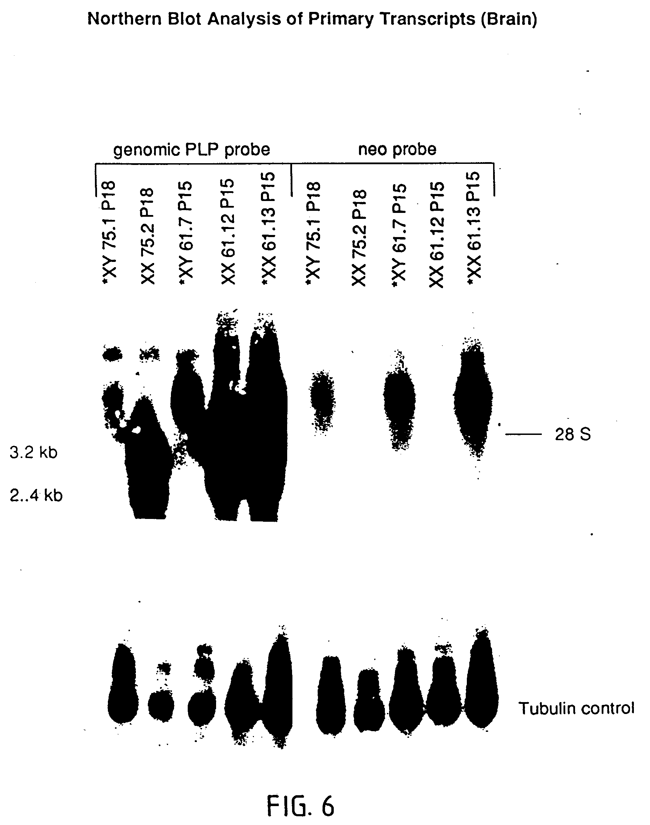

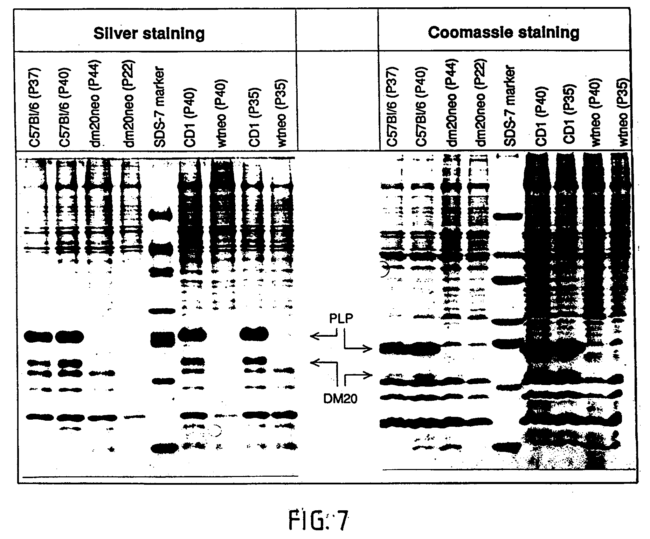

On the basis of the correct DNA stucture of the plp⁺/dm20⁺/asneo and plp⁻/ dm20⁺/asneo replacements at the PLP locus we expected the spliced PLP and DM20 mRNA transcripts in Northern blot analysis. Figures 5 shows the Northern blot of total RNA of brains of 15 to 20 day old wild type, heterozygous and homozygous mutant litter mates of line 75 (plp⁺/dm20⁺/asneo) and line 61 (plp⁻/dm20⁺/asneo) hybridized with a PLP cDNA (-74 to 298) probe. RNA of homozygous mutant mice (*XY) completely lack the three typical PLP mRNAs of 1.6, 2.4, and 3.2 kb. The three different transcripts result from the use of three polyadenylation signals (Schaich et al., 1986). Total RNA from homozygous (*XY) and heterozygous (*XX) mutant mice, however, gives weak signals in the >5kb range, Figure 6, indicating unspliced precursor RNA when probed with a 800 bp PstI fragment of the neo-cassette or when probed with an intron II and III-containing genomic PLP probe, Figure 6. Currently we are investigating the precise nature of these transcripts, whether they are unspliced sense transcripts generated by the PLP promoter or whether they are antisense transcripts driven by the tk-promoter of the neo gene and readthrough of its polyadenylation signal.

Proteins of total brain, purified myelin and chloroform-methanol extract of myelin of wild type, hetero- and homozygous mutant mice were analyzed by SDS-polyacrylamide gel electrophoresis and compared with bovine myelin. As expected from the Northern blot analysis PLP and DM20 bands were absent in the samples of homozygous transgenic animals of line 61.

The results with control line 75 were identical. No PLP or DM20-specific signals could be detected in mutant mice by Western blot analysis using PLP-specific peptide antibodies which probed epitopes Arg⁹⁷ to Leu¹¹², Gly¹¹⁹ to Gly¹²⁷, and Thr²⁶¹ to Phe²⁷⁶ of PLP.

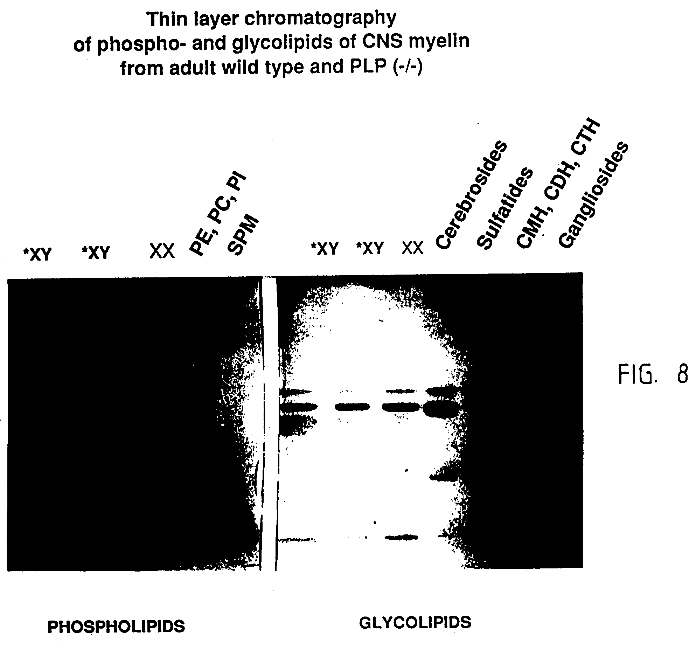

The composition of phospho- and glycosphingolipids in CNS myelin was analyzed by thin-layer chromatography. Neither the phospholipid nor glycosphingolipid pattern was visibly affected by the mutation, Figure 8.

### Electron Microscopy

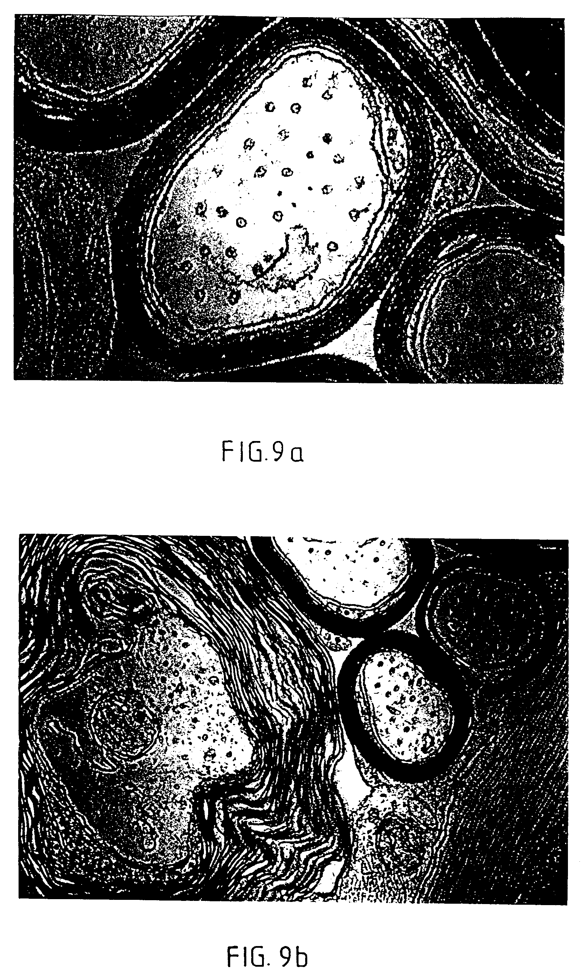

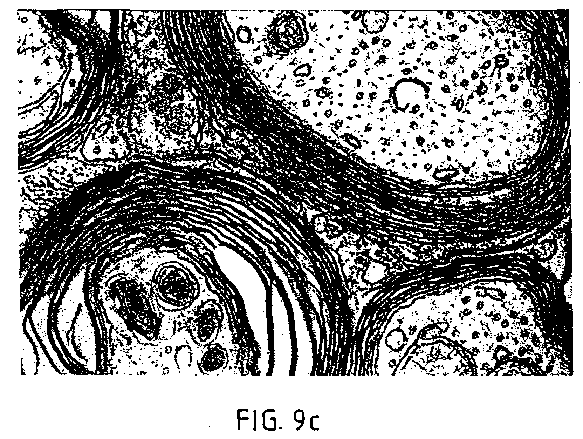

To assess the morphological consequences for wrapping and compaction of the myelin membrane devoid of its main integral membrane proteins PLP and DM20, we investigated the optical nerve of wild type, heterozygous and homozygous mutant plp⁻/dm20⁺/asneo mice at the age of 20 days by electron microscopy, Figure 10A-C. The results were stunning, showing a completely disordered myelin in homozygous mutants, with plasmamembrane processes of oligodendrocytes only loosely wrapping larger axons whereas smaller axons remain unmyelinated. The loosely compacted myelin shows a complete absence of the intraperiod dense line, which is formed normally by compaction of the extracytoplasmic membrane surfaces, Figure 9C.

Heterozygous females show a cellular mosaicism, due to random X-inactivation during Barr-body formation. We find normally myelinated axons with compacted intraperiod dense lines in the vicinity of loosely wrapped axons. Since PLP is specific for myelinating oligodendrocytes we expected normal myelin ultrastructure in peripheral nerves. As expected, myelin sheaths of axons of peripheral nerve of homozygous mutant mice were indistinguishable from those observed in wild type mice.

### Electrophysiological data and behaviour

In spite of the severe structural effects on myelin compaction, the transgenic mice show no gross neurological deficits or abnormalities in behaviour. The animals have normal fertility, so that the mutation could be bred to homozygozity. To date the oldest mutant animals have reached an age of seven months and remain healthy. To elucidate possible changes in electrical nerve conductance we measured the velocity of conductance in optical nerves freshly prepared from wild type, heterozygous and homozygous mutant mice. We found a reduction in conductance velocity in homozygous mutants by one half and in heterozygous mutants by one third as compared with wild type controls.

### Experimental Procedures

### Construction of Targeting Vectors

To construct a vector for targeting the PLP gene we first subcloned a 6.7 kb DNA fragment encompassing exons I through V derived from a genomic Balb/c mouse library into the polycloning site of the pUC19 vector. To introduce specific mutations into exon III of the PLP gene mutagenesis has been performed by overlapping PCR. For the introduction of the 105 bp DM 20 deletion into exon III primer ExIIS 5'-GTTTGTTAGAGTGCTGTGCTAGATGTCTGGT-3', a sense primer located at the 5' end of exon II, and primer DELA 5'-aatcctgaggatgatcacCGTTGCGCTCAGGCCCTT-3', an antisense primer containing the DM 20 deletion, have been used for the first round of 5' amplification giving rise to a 1.3 kb segment. Primer ExIVA 5'-GCCATACAACAGTCAGGGCATAGGTGATGC-3', an antisense primer located at the 5' end of exon 4, and primer DELS 5'-AAGGGCCTGAGCGCAACGgtgatcatcctcaggatt-3', a sense primer containing the DM 20 deletion, have been used for the first round of 3' amplification giving rise to a 1.1 kb segment. These two gel-purified fragments have been combined for the overlapping PCR reaction using primers ExIIS and ExIVA for amplification of the 2.4 kb overlap product. The final product was gel-purified, digested with HindIII and BamHI to clone exon III with its surrounding intron regions into pUC19. The sequence has been controlled by sequencing with a T7 sequencing kit (Pharmacia).

Following the same strategy another exon III mutation has been subcloned. The PLP⁺/DM20⁻ mutation containing a two base exchange in the consensus sequence at the alternative splice signal within exon III thus prohibiting the formation of DM 20 transcripts has been created by use of the mutagenesis primers MUTS 5'-GCCTGAGCGCAACCGTCACAGGGGGCCAGA-3' and MUTA 5'-TCTGGCCCCCTGTGACGGTTGCGCTCAGGC-3'.

PCR cycling was for 1.5 min at 93 °C, 2 min at 68 °C, and 2 min at 72 °C for 15 cycles with 100ng template DNA, 100 pmol of each primer, 200 µM dNTPs, 10 mM Tris-HCl (pH 8.3), 50 mM KCl, 1.5 mM MgCl₂, 0.1 mg/ml gelatin and 1.5 U of Taq polymerase (BRL) in a final volume of 100 µl.

The amplification products have been electrophoresed and visualised on 1% agarose gels by ethidium bromide staining and eluted using the Quiaex Gel Extraction Kit (Quiagen).

The final gene targeting constructs have been cloned in five steps. First a unique HpaI cleavage site was introduced by the use of 5' sequence identical HpaI-specific oligonucleotide linkers 5'-CATGGAATGCTTTCCCTGGCAAGGTTAAC-3' and 5'-CATGGTTAACCTTGCCAGGGAAAGCATTC-3' into the NcoI site of exon V which was subcloned as a 1.8 kb BamHI-fragment encompassing exons IV and V into phagemide pEMBL19. This way HpaI cleavage creates blunt 3' ends of the targeting constructs, with 100% PLP sequence identity. The product pEMBLplp1.8HpaI was cut with BamHI, the 1.8 kb PLP fragment eluted and cloned in sense orientation into the BamHI site of the tk-cassette containing vector pIC19r-MCI-tk after a fill-in reaction (Maniatis et al., 1982) had been performed to destroy these BamHI sites after ligation. The resulting construct pPLP45^{h}tk was cut with SalI and expanded with a SalI-XhoI neo-cassette from pMCIneopA (Stratagene) in antisense orientation within intron III giving rise to vector pPLP45^{h}asneotk. This vector was used as a basal construct to be expanded by three different 1.2 kb BamHI/HindIII fragments containing the two mutated forms of exon III, and wt-exon III as a control. The resulting vector pPLPX35^{h}asneotk (X standing for either wt, Del, or Mut) was linearized with the unique HindIII site, and the exon II-containing HindIII fragment was added. The end product pPLPX25^{h}asneotk can be linearized with HpaI only to be used as an insertion vector, HpaI/ClaI as a replacement vector, and SmaI as a PCR mock construct.

### Gene Targeting in ES Cells

C57BL/6J and CD1 mice were obtained from Jackson Laboratory and Charles River (Sulzfeld). E14 ES cells (passages 10 to 15) were grown to 90% confluence on mitomycin C-treated G418 resistant mouse embryonic fibroblast feeder layers (Robertson, 1987) in Dulbecco's modified Eagle's medium containing 15% fetal calf serum, 0.1 mM 2-mercaptoethanol, and 500 U LIF (Esgro) per ml at 37 ᵢC, 5% CO₂. A total of 11 electroporations were performed using 10⁷ cells per experiment in 0.8 ml phosphate buffered saline and 20 µg of HpaI/ClaI-linearized replacement vector DNA in a 0.4 cm cuvette of a Bio-Rad gene pulser set at 220 V, 500 µF. Cells from each cuvette were then distributed equally on six 100 mm feeder plates. G418 (250 µg effective concentration, Sigma) and gancyclovir (2 µM) were added on four plates for double selection after 24 h. One control plate was selected with G418 only, the other control plate was fed with normal culture medium. After 8 to 10 days resistant clones were picked into 24 microwell plates coated with mouse embryonic fibroblast feeder cells. After expansion each clone was split into two wells. One of these samples was frozen, while the other one was expanded on a gelatinized well for DNA analysis. On average 20% to 33% of the G418-resistant colonies were also resistant to gancyclovir, and 2% to 3% of these were homologous recombinants. In the PLP⁻/ DM20⁺ targeting experiment (deletion) 5 out of 6 clones carried a coconversion of the desired mutation with the homologously integrated neo-box whereas in the PLP⁺/DM20⁻ (two mismatches) targeting experiment the ratio was only one coconversion out of seven homologously recombined clones. The targeted clones have been checked for ECMA7 and TROMA1 immunofluorescence staining and for their karyotype prior to injections.

### ES Cell DNA Analysis

After reaching 75% confluence cells were washed with phosphate buffered saline and 500 µl of lysis buffer (50 mM Tris-HCl [pH 7.5], 100 mM NaCl, 50 mM EDTA, 1% SDS and 400 µg/ml proteinase K) was added to each well. The plates were incubated over night at 37 °C.The lysates were transferred into 1.5 ml Eppendorf tubes and precipitated after the addition of 500 µl isopropanol. The pellet was washed once with 70% ethanol, dried and resuspended over night at 37 °C on a rocking platform in 50 µl TE. One third of the DNA yield of three clones was pooled for Southern Blot analysis using an EcoRI digestion giving rise to the indicative fragments of 7 kb (non-targeted) and 4.5 kb (targeted) resulting from an additional EcoRI site contained within the neo-box. The external ³²P-labeled PLP probes plp56 and plp67 (not contained within the vector) were used for hybridization. The digests were fractionated on 0.7% agarose gels and transferred onto a GeneScreenPlus® membrane according to the manufacturer's directions. Positive pools were analyzed for their EcoRI restriction pattern of the individual clones. Positive clones were checked by BglII restriction and exon III probe hybridization for the presence of the DM 20 deletion. Additionally clones were checked by PCR using the primers neoATG (neo-specific antisense primer lying on the ATG start codon of the neo gene) 5'-AATCCATCTTGTTCAATGGCCGATCCCAT-3' and HRA (PLP antisense primer located on genomic DNA, downstream of the 3' end of the targeting construct), 5'-TCAGCTGTTTTGCAGATGGACAGAAGGTTGG-3', specific for a homologous recombination event. PCR was performed in 67 mM Tris-HCl pH 8.3, 16.6 mM NH₄SO₄, 0.17 mg/ml BSA, 10 mM 2-mercaptoethanol, 1 mM MgCl₂, 5% DMSO, using 5 pmol of each primer, 10 mM of each of the dNTPs and 1.5 U of Taq polymerase (BRL), cycling for 1 min at 92°C, for 1 min at 50 °C, and for 3 min 30 sec at 72 °C for a total of 35 cycles. The existence of the specific mutations was checked, using the same conditions with primers ExIIS and SauA 5'-GAGGACCCAACCTTGGGTGGGGATCAGGCT-3' to amplify exon III, giving a 1.2 kb band in case of PLP and a 1.1 kb band in case of the DM 20 deletion. The PLP⁺/DM20⁻ splice site mutation was checked with the allele specific primer DM-5'-ATCTGCGGCAAGGGCCTGAGCGCAACGTC-3' in conjunction with ExIVA or SauA. This way five correct plp⁻/dm20⁺/asneo clones and one plp⁺/dm20⁻/asneo clone could be identified in addition to a control clone plp⁺/dm20⁺/asneo.

### Blastocyst Injections

Blastocysts were flushed at 3.5 days postcoitum from the uterine horns of super-ovulated C57Bl/6J and CD1 females in Dulbecco's modified Eagle's medium containing 10% fetal calf serum and 50 mM HEPES. 10 to 20 cells of targeted clones were injected into each blastocoel and groups of 5 to 14 blastocysts were transferred into pseudopregnant females as described (Bradley et al., 1984). Two of the injected clones yielded one germ line chimera each.

### Genotyping

For genotyping of animals by Southern analysis and PCR, DNA was prepared from the tail tips of 2 week old mice as described (Hogan, 1986). As probes we used the genomic fragments plp56 and plp67 lying downstream of the 3' end of the targeting constructs and a 1.2 kb BamHI-HindIII fragment specific for exon III. As PCR primers we used the combinations NeoATG with HRA and ExIIS with SauA (Figure 2). After genotyping, mice were crossed specifically to obtain homozygous transgenic lines.

### Expression Analysis

### RNA analysis

Total RNA of 15 to 20 day old mouse brain was extracted as described (Chomczynski and Sacchi, 1987). 15 µg of RNA was separated on 1.5% agaroseformaldehyde gels and blotted onto nitrocellulose (Schleicher and Schüll) with 20 x SSC. After baking for 2 hours at 80 °C the membrane was prehybridized in 50 mM phosphate buffer, pH 6.8,5 x SSC, 1 x Denhardt's, 50% formamide, and 100 µg/ml salmon sperm DNA for 4 hours at 42 °C. After that time the membrane was hybridized in fresh buffer containing 10% dextrane sulphate and 2 x 10⁵ cpm/ml ³²P-labeled probe over night at 42 °C. The membrane was washed 4 times 5 min. in 2 x SSC, 0.1% SDS at room temperature, and 2 times 15 min. in 0.1 x SSC, 0.1% SDS at 50 °C.

### Protein Analysis

Total brain extract, total brain myelin and chloroform/methanol (1:1 vol/vol) extracts of myelin (Norton, 1973) were seperated on 15% SDS-PAGE and either stained with Coomassie or by silver staining.

### Lipid Analysis

Chloroform methanol (2:1 v/v) extracts of total brain or density gradient purified myelin were separated by thin layer chromatography (solvent system:chloroform:methanol:water 60:25:4) on HPTLC Precoated Plates, Silica Gel (Merck, Darmstadt). Phospholipids were stained with Zinzadze reagent (Dittmer and Lester, 1964), and glycosphingolipids visualized with α-naphthol-H₂SO₄ in methanol-water (1:1,v/v).

### Histological Analysis

### Electron Microscopy

Mice were anaesthesized with Nembutal and perfused with 6% glutaraldehyde via the left cardiac ventricle. The optical nerve was removed between the eyeball and the orbital fissure, postfixed in 1% phosphate buffered OsO₄ in 0.1 M sucrose and embedded in Epon 812. Ultrathin cross sections of the optical nerve were contrasted with uranylacetate and lead citrate and examined.

### Legends to Figures

### Figure 1

Topology of PLP and DM20 in the Lipid Bilayer of CNS Myelin Membrane.
Amino acid residues 115 to 150 oriented toward the cytosolic cleft are missing in DM20 due to alternative splicing. This domain containing two acylation sites is marked in black. PLP domains are assigned to the seven PLP exons.

### Figure 2

### PLP Gene, Targeting Construct and Homologous Recombination Event of the PLP Gene

(A) Restriction map of the PLP gene. Numbered boxes indicate exons, intron sequences are indicated with a solid line. E, H, and B represent cleavage sites from EcoRI, HindIII, and BglII, respectively.
(B) Map of the PLP targeting constructs pPLPX25^{h} asneotk. The mutated exon III is indicated with a solid box and the tk and neo-genes are indicated, with their respective orientation marked by an arrow. Note that the neo-cassette is inserted in antisense orientation within intron III of the PLP gene.
(C) Expected and observed replacement event. The diagnostic restriction polymorphisms and PCR products are indicated together with the location of external and internal probes (thick bars) for Southern blot analysis and the location of analytical PCR primers (arrows) with their respective amplification products.

### Figure 3

### Southern Blot Analysis of ES Cell Pools and Individual Clones

BglII restriction of individual clones, exon III probe. The probe indicates the 105 bp deletion of the PLP⁻/DM 20⁺ replacement event in homologous recombination, as a 600 bp BglII fragment in comparison to the 700 bp wt-fragment present in heterologous clones, and wt ES cells.

### Figure 4

### Analysis of F2-Generation Offspring of Transgenic Line 61 (plp⁻/dm 20⁺/neo)

(A) Southern hybridization of a HindIII-digested DNA obtained from the tail tips of two-week-old pups. The external probes plp56 and plp67 were used as probes to detect the 6 kb HindIII fragment present in wt XX and XY, and heterozygous *XX female mice, and the 7 kb fragment (addition of the1.1 kb neo-cassette) present in heterozygous *XX and homozygous mutant *XY mice.
(B) PCR analysis using primers ExIIS/SauA located on either side of the DM20 deletion giving rise to a 1.2 kb band for the wt-allele present in XY, XX, and *XX mice and a 1.1 kb band present in the heterozygous *XX and homozygous mutant *XY mice.

### Figure 5

### Northern Blot Analysis of Total Mouse Brain RNA.

Total RNAs from plp⁺/dm20⁺/asneo mice (# 75.1,2,4,7) and plp⁻/dm20⁺/asneo mice (#61.7,8,10,12,13,16) heterozygous and homozygous for the respective alleles where size-fractioned through a 1.5% agarose-formaldehyde gel, capillary blotted to a nitrocellulose membrane and hybridized to a ³²P-labelled rat PLP cDNA probe (-74 to 298 bp). Films were exposed for 24 hours at -70ᵢC using intensifying screens. Blots were reprobed with α-tubulin as a control for RNA quantities in lanes (bottom). The age of the mice sacrificed is indicated with P15, P18, and P20 for 15, 18, and 20 days after birth.

### Figure 6

### Northern Blot Analysis of Total Mouse Brain RNA.

The RNA is probed with a genomic intron II/III probe (left) or with a neo cassette-probe (right). The longer exposure time (72 hours) of the autoradiography shows the additional >5 kb unspliced transcripts present in mice, containing the mutated X-chromosome, containing genomic intron sequences and the transcribed neo cassette, indicating a splicing defect in these mice. Wild type and heterozygous mutant mice show the normally spliced PLP transcripts of 3.2,2.4, and 1.6 kb. The blots have been reprobed with an α-tubulin-fragment for quantification of the RNA amounts applied to the lanes.

### Figure 7

### Protein Analysis of Wildtype and Transgenic PLP/DM20 Deficient Mice

Silver stained SDS-PAGE (15%) analysis of CNS myelin protein wild type C57Bl/6 and CD1, and homozygous mutant dm20/neo and plp/dm20/neo (wt/neo). Isolated myelin is completely devoid of PLP and DM 20 in homozygous PLP/DM20 deficient mutants (dm20/neo and plp/dm20/neo (wt/neo). The age of the sacrificed animals is indicated in postnatal days.

### Figure 8

### Lipid Analysis of Wildtype and Transgenic PLP-Deficient Mice.

Thin layer chromatography of phospho- and glycolipids of CNS myelin from adult wild type and PLP-deficient mice, line 61,plp⁻/dm20⁺/asneo separated in chloroform/methanol/water 65/25/4.
(A) Phospholipid staining with Zinzadze reagent (Dittmer and Lester, 1964). XX wildtype,*XX heterozygous, and *XY homozygous mutant show an identical pattern of PE (phosphatidylethanolamine), PC (Phosphatidylcholine), PI (Phosphatidylinositol), and SPM (sphingomyelin).
(B) Pattern of glycosphingolipids stained with anthron-H₂SO₄. Cerebrosides and sulfatides from bovine brain are added as standards.

### Figure 9

### Electron Micrographs of Cross Sections of the Optic Nerve of plp⁻/dm20⁺/asneo Mice (age 29 days)

(A) Wild type (XY): only myelinated axons are visible in this micrograph. Note the overall compact appearance of the myelin sheaths, showing regularly compaction visible as major and intraperiod dense lines (1:16 000).
(B) Heterozygous mutant (*XX): mosaicism of normally myelinating and loosely wrapping oligodendrocytes due to random X-inactivation (1:16 000).
(B*) Same as (B), magnification 1:48 000.
(C) Homozygous mutant (*XY): non-compacted myelin sheaths around large diameter axons. Note the absence of the intraperiod dense line. Small diameter axons remain unmyelinated (1:16 000).
(C*) same as (C), magnification 1:48 000.

## Claims

1. A transgenic non-human mamalian which is unable to form proteolipid protein for the myelin compaction in central nervous systems.

2. A transgenic non-human mamalian according to Claim 1, wherein the formation of proteolipid protein is supressed by the presence of antisense RNA.


SEQUENCE LISTING

(1) GENERAL INFORMATION:

(i) APPLICANT:
(A) NAME: Bayer AG
(B) STREET: Bayerwerk
(C) CITY: Leverkusen
(E) COUNTRY: Germany
(F) POSTAL CODE (ZIP): D-51368
(G) TELEPHONE: 0214/3061455
(H) TELEFAX: 0214/303482

(ii) TITLE OF INVENTION: Trangenic Animals Lacking Proteolipid
Protein and Method of Making Such Animal

(iii) NUMBER OF SEQUENCES: 12

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(2) INFORMATION FOR SEQ ID NO: 1:

(i) SEQUENCE CHARACTERISTICS:
(A) LENGTH: 31 base pairs
(B) TYPE: nucleic acid
(C) STRANDEDNESS: single

(D) TOPOLOGY: linear

(ii) MOLECULE TYPE: cDNA

(iii) HYPOTHETICAL: NO

(iii) ANTI-SENSE: NO

(vi) ORIGINAL SOURCE:

(C) INDIVIDUAL ISOLATE: Synthetic

(xi) SEQUENCE DESCRIPTION: SEQ ID NO: 1:

GTTTGTTAGA GTGCTGTGCT AGATGTCTGG T 31

(2) INFORMATION FOR SEQ ID NO: 2:

(i) SEQUENCE CHARACTERISTICS:
(A) LENGTH: 36 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: YES

(vi) ORIGINAL SOURCE:
(C) INDIVIDUAL ISOLATE: Synthetic

(xi) SEQUENCE DESCRIPTION: SEQ ID NO: 2:

AATCCTGAGG ATGATCACCG TTGCGCTCAG GCCCTT 36

(2) INFORMATION FOR SEQ ID NO: 3:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(vi) ORIGINAL SOURCE:
(C) INDIVIDUAL ISOLATE: Synthetic

(xi) SEQUENCE DESCRIPTION: SEQ ID NO: 3:

GCCATACAAC AGTCAGGGCA TAGGTGATGC 30

(2) INFORMATION FOR SEQ ID NO: 4:

(i) SEQUENCE CHARACTERISTICS:
(A) LENGTH: 36 base pairs
(B) TYPE: nucleic acid

(C) STRANDEDNESS: single
(D) TOPOLOGY: linear

(ii) MOLECULE TYPE: cDNA

(iii) HYPOTHETICAL: NO

(iii) ANTI-SENSE: YES

(vi) ORIGINAL SOURCE:
(C) INDIVIDUAL ISOLATE: Synthetic

(xi) SEQUENCE DESCRIPTION: SEQ ID NO: 4:

AAGGGCCTGA GCGCAACGGT GATCATCCTC AGGATT 36

(2) INFORMATION FOR SEQ ID NO: 5:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: cDNA

(iii) HYPOTHETICAL: YES

(iii) ANTI-SENSE: NO

(vi) ORIGINAL SOURCE:

(C) INDIVIDUAL ISOLATE: Synthetic

(xi) SEQUENCE DESCRIPTION: SEQ ID NO: 5:

GCCTGAGCGC AACCGTCACA GGGGGCCAGA 30

(2) INFORMATION FOR SEQ ID NO: 6:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(vi) ORIGINAL SOURCE:
(C) INDIVIDUAL ISOLATE: Synthetic

(xi) SEQUENCE DESCRIPTION: SEQ ID NO: 6:

TCTGGCCCCC TGTGACGGTT GCGCTCAGGC 30

(2) INFORMATION FOR SEQ ID NO: 7:

(i) SEQUENCE CHARACTERISTICS:

(A) LENGTH: 29 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(vi) ORIGINAL SOURCE:
(C) NDIVIDUAL ISOLATE: Synthetic

(xi) SEQUENCE DESCRIPTION: SEQ ID NO: 7:

CATGGAATGC TTTCCCTGGC AAGGTTAAC 29

(2) INFORMATION FOR SEQ ID NO: 8:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(vi) ORIGINAL SOURCE:
(C) INDIVIDUAL ISOLATE: Synthetic

(xi) SEQUENCE DESCRIPTION: SEQ ID NO: 8:

CATGGTTAAC CTTGCCAGGG AAAGCATTC 29

(2) INFORMATION FOR SEQ ID NO: 9:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: YES

(vi) ORIGINAL SOURCE:
(C) INDIVIDUAL ISOLATE: Synthetic

(xi) SEQUENCE DESCRIPTION: SEQ ID NO: 9:

AATCCATCTT GTTCAATGGC CGATCCCAT 29

(2) INFORMATION FOR SEQ ID NO: 10:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: YES

(vi) ORIGINAL SOURCE:
(C) INDIVIDUAL ISOLATE: Synthetic

(xi) SEQUENCE DESCRIPTION: SEQ ID NO: 10:

TCAGCTGTTT TGCAGATGGA CAGAAGGTTG G 31

(2) INFORMATION FOR SEQ ID NO: 11:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(vi) ORIGINAL SOURCE:
(C) INDIVIDUAL ISOLATE: Synthetic

(xi) SEQUENCE DESCRIPTION: SEQ ID NO: 11:

GAGGACCCAA CCTTGGGTGG GGATCAGGCT 30

(2) INFORMATION FOR SEQ ID NO: 12:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(vi) ORIGINAL SOURCE:
(C) INDIVIDUAL ISOLATE: Synthetic

(xi) SEQUENCE DESCRIPTION: SEQ ID NO: 12:

ATCTGCGGCA AGGGCCTGAG CGCAACGTC 29